(19) Europäisches Patentamt / European Patent Office / Office européen des brevets

(11) **EP 2 717 938 B1**

(12) **EUROPÄISCHE PATENTSCHRIFT**

(45) Veröffentlichungstag und Bekanntmachung des Hinweises auf die Patenterteilung:
**19.08.2015 Patentblatt 2015/34**

(21) Anmeldenummer: **12727312.6**

(22) Anmeldetag: **06.06.2012**

(51) Int Cl.:
*A61M 1/16* *(2006.01)*

(86) Internationale Anmeldenummer:
**PCT/EP2012/002415**

(87) Internationale Veröffentlichungsnummer:
**WO 2012/167929 (13.12.2012 Gazette 2012/50)**

(54) **VERFAHREN UND VORRICHTUNG ZUM BESTIMMEN MINDESTENS EINES VOM ABSOLUTDRUCK ABHÄNGIGEN BETRIEBSPARAMETERS EINER VORRICHTUNG ZUR EXTRAKORPORALEN BLUTBEHANDLUNG, VORRICHTUNG ZUR EXTRAKORPORALEN BLUTBEHANDLUNG**

METHOD AND DEVICE FOR DETERMINING AT LEAST ONE ABSOLUTE-PRESSURE-DEPENDENT OPERATING PARAMETER OF A DEVICE FOR EXTRACORPOREAL BLOOD TREATMENT, DEVICE FOR EXTRACORPOREAL BLOOD TREATMENT

PROCÉDÉ ET DISPOSITIF DE DÉTERMINATION D'AU MOINS UN PARAMÈTRE DE FONCTIONNEMENT D'UN DISPOSITIF DE TRAITEMENT EXTRACORPOREL DU SANG DÉPENDANT DE LA PRESSION ABSOLUE, DISPOSITIF DE TRAITEMENT EXTRACORPOREL DU SANG

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **09.06.2011 DE 102011106111**
**09.06.2011 US 201161495060 P**

(43) Veröffentlichungstag der Anmeldung:
**16.04.2014 Patentblatt 2014/16**

(73) Patentinhaber: **Fresenius Medical Care Deutschland GmbH**
**61352 Bad Homburg (DE)**

(72) Erfinder: **GAGEL, Alfred**
**96123 Litzendorf (DE)**

(74) Vertreter: **Ziermann, Oliver**
**Fresenius Medical Care AG & Co. KGaA**
**Global Patents & IP**
**Siemensstraße 21**
**61352 Bad Homburg (DE)**

(56) Entgegenhaltungen:
**DE-A1- 19 919 572     US-A- 4 086 653**
**US-A1- 2006 283 450     US-A1- 2009 099 498**
**US-A1- 2009 124 963**

EP 2 717 938 B1

**Beschreibung**

Die Erfindung betrifft ein Verfahren und eine Vorrichtung zum Bestimmen mindestens eines vom Absolutdruck abhängigen Betriebsparameters einer Vorrichtung zur extrakorporalen Blutbehandlung. Nachfolgend wird zur Vereinfachung der Begriff Blutbehandlungs-Vorrichtung für alle Vorrichtungen zur extrakorporalen Blutbehandlung verwendet.

Es sind verschiedene Arten von Blutbehandlungsvorrichtungen bekannt. Zu den bekannten Blutbehandlungsvorrichtungen zählen beispielsweise die Vorrichtungen zur Hämodialyse, Hämofiltration und Hämodiafiltration. Während der extrakorporalen Blutbehandlung strömt das Blut in einem extrakorporalen Blutkreislauf durch eine Blutbehandlungseinheit. Bei den Vorrichtungen zur Hämodialyse, Hämofiltration und Hämodiafiltration ist die Blutbehandlungseinheit ein Dialysator oder Filter, der schematisch betrachtet durch eine semipermeable Membran in eine Blutkammer und eine Dialysierflüssigkeitskammer getrennt ist. Während der Blutbehandlung mittels Hämodialyse oder Hämodiafiltration strömt das Blut durch die Blutkammer, während eine Dialysierflüssigkeit durch die Dialysierflüssigkeitskammer strömt.

Die US 2009/0124963 A1 beschreibt ein System und ein Verfahren zum Messen und Bilanzieren der Flüsse von Dialysat und Ultrafiltrat bei der Nierenersatztherapie mittels Druckmessungen in Behältern auf der Grundlage des Gesetzes von Boyle.

Die US 2009/0099498 A1 beschreibt ein Herz-Lungen-System, von dem das Bestimmen des Volumens einer Kammer mittels Druckmessungen auf der Grundlage des Gesetzes von Boyle bekannt ist.

Die DE 199 19 572 A1 beschreibt ein Verfahren und eine Vorrichtung zur Bestimmung des Gasanteils in einem Gemisch aus Gas und Flüssigkeit, wobei das Gemisch aus Gas und Flüssigkeit mittels einer Pumpe sowohl gefördert als auch einer Volumen- und Druckänderung unterworfen wird und unter Berücksichtigung der Betriebsparameter der Pumpe und Druckmessungen auf der Grundlage des Gesetzes von Boyle der Gasanteil bestimmt wird.

Die Bereitstellung frischer Dialysierflüssigkeit kann durch ein Dialysierflüssigkeitssystem erfolgen, das in die Vorrichtung zur extrakorporalen Blutbehandlung integriert ist. In dem Dialysierflüssigkeitssystem kann Reinwasser z.B. aus einer Umkehrosmose zugeführt werden, das zunächst entgast und dann mit Flüssigkonzentraten zur Herstellung von frischer Dialysierflüssigkeit vermischt wird. Die Vermischung kann beispielsweise durch Zugabe der Flüssigkonzentrate an getrennten Zugabestellen in die Reinwasserleitung und nachfolgende vollständige Vermischung in einer Mischkammer oder durch direkte Zugabe der Flüssigkonzentrate über getrennte Zugabestellen in eine Mischkammer erfolgen. Die frische Dialysierflüssigkeit durchströmt zunächst ein Bilanziersystem und wird danach durch die Dialysierkammer des Dialysators geleitet. Dabei wird die frische Dialysierflüssigkeit mit Wasser und Inhaltsstoffen des Bluts beladen und wird dadurch zur gebrauchten Dialysierflüssigkeit. Nach dem Verlassen des Dialysators wird die gebrauchte Dialysierflüssigkeit durch das Bilanziersystem geleitet. Dabei wird eine Differenz zwischen dem Volumen der frischen Dialysierflüssigkeit und der gebrauchten Dialysierflüssigkeit ermittelt.

Die Mischkammer weist zuführende Flüssigkeitsleitungen und abführende Flüssigkeitsleitungen auf. Der Mischkammer kann unvollständig vorgemischtes Gemisch aus entgastem Reinwasser und Flüssigkonzentraten zugeführt werden. In der Mischkammer erfolgt die vollständige Vermischung. Frische Dialysierflüssigkeit wird aus der Mischkammer durch eine Dialysierflüssigkeitsleitung abgeführt. Die Förderung von Reinwasser, Flüssigkonzentraten und Dialysierflüssigkeit in den Leitungen erfolgt durch Pumpen. Die Entgasung des Reinwassers erfolgt durch Erzeugen eines Unterdrucks mittels einer Entgasungspumpe in die Reinwasserleitung stromaufwärts der Mischkammer. Die Förderung der Flüssigkonzentrate erfolgt durch Dosierpumpen stromaufwärts der Mischkammer. Die Förderung der Dialysierflüssigkeit erfolgt durch eine Dialysierflüssigkeitspumpe in der Dialysierflüssigkeitsleitung. Mit der Dialysierflüssigkeitsleitung vor dem Dialysator und mit der Dialysierflüssigkeitsleitung nach dem Dialysator können weitere Pumpen in fluidführender Verbindung stehen, wie beispielsweise eine Flusspumpe in der Dialysierflüssigkeitsleitung und eine Ultrafiltrationspumpe.

Bei den gattungsgemäßen Vorrichtungen zur extrakorporalen Blutbehandlung werden zur Messung der Drücke üblicherweise kostengünstige Relativdrucksensoren eingesetzt. Die Drücke innerhalb des Dialysierflüssigkeitssystems werden deshalb üblicherweise in Bezug auf den Umgebungsdruck eingestellt. Die gattungsgemäßen Vorrichtungen zur extrakorporalen Blutbehandlung verfügen deshalb üblicherweise nicht über einen integrierten Absolutdruckmesser.

Die Einstellungen vom Absolutdruck abhängiger Betriebsparameter kann bei bekannten Vorrichtungen zur extrakorporalen Blutbehandlung entweder von einem Servicetechniker unter Verwendung eines externen Absolutdruckmessgeräts durchgeführt werden, oder es werden in der zentralen Steuereinheit Näherungswerte zur Auswahl hinterlegt.

Die Einstellung der vom Absolutdruck abhängigen Betriebsparameter erfolgt beispielsweise bei der Aufstellung einer Blutbehandlungsvorrichtung in einer ausgewählten geografischen Region durch einen Servicetechniker, der dazu ein externes Absolutdruckmessgerät mit sich führen kann.

Beispielsweise wird nach dem Stand der Technik bei der Aufstellung eines Blutbehandlungsgeräts die Höhe des Aufstellortes durch die Auswahl von Wertebereichen eingestellt, und es werden für jeden Wertebereich mittlere Werte für die vom Absolutdruck abhängigen Betriebsparameter vorgegeben. Beispielsweise werden Mittelwerte der vom Absolutdruck abhängigen Betriebsparameter für folgende Wertebereiche in Abhängigkeit die Höhe des Aufstellortes über dem Meeresspiegel vorgegeben:

- Aufstellhöhe kleiner als 800 m über N.N. (N.N. = Normal Null des Meeres-spiegels),
- Aufstellhöhe zwischen als 800 m über N.N. und 1.400 m über N.N.,
- Aufstellhöhe über 1.400 m N.N. bis 2.000 m über N.N.,
- Aufstellhöhe über 2.000 m über N.N..

Die Höhe des Aufstellortes kann in der Regel ohne weitere Hilfsmittel grob abgeschätzt werden. Nach der Auswahl eines Wertebereiches werden die für den Wertebereich vorgegebenen Betriebsparameter eingestellt.

Die bekannten Methoden zur Einstellung vom Absolutdruck abhängiger Betriebsparameter an Blutbehandlungsvorrichtungen sind mit Nachteilen verbunden, weil sie entweder aufwändig und kaum automatisierbar oder aber ungenau und zudem von der Zuverlässigkeit einer fehleranfälligen Benutzereingabe abhängig sind.

Beispiele für solche vom Absolutdruck abhängigen Betriebsparameter sind der Entgasungsdruck und die Siedetemperatur. Der Absolutdruck kann auch selbst ein Betriebsparameter einer Vorrichtung zur extrakorporalen Blutbehandlung sein.

Der lokale Umgebungsdruck kann je nach geografischer Lage und Wetterlage beispielsweise zwischen 700 hPa und 1.060 hPa liegen. Auch bei wetterbedingten Änderungen des Absolutdrucks wäre eine automatische Anpassung der vom Absolutdruck abhängigen Betriebsparameter wünschenswert.

Die Einstellung des Entgasungsdrucks ist für eine sichere und zuverlässige Entgasung des Reinwassers erforderlich. Das Lösungsverhalten von Gasen in Flüssigkeiten ist vom Absolutdruck abhängig. Dabei nimmt die Sättigungskonzentration mit abnehmendem Absolutdruck ab. Es wird bei gattungsgemäßen Blutbehandlungsvorrichtungen ein absoluter Entgasungsdruck von rund 150 hPa bezogen auf das Vakuum angestrebt. Der Entgasungsdruck wird durch eine Entgasungspumpe und eine Drossel in der Reinwasserleitung des Dialysierflüssigkeitsystems erzeugt. Ohne Absolutdruckmessung kann der Entgasungsdruck nur näherungsweise festgestellt werden. Für eine sichere und zuverlässige Entgasung ist die möglichst genaue Einstellung des Entgasungsdrucks wünschenswert.

Die Siedetemperatur wird benötigt, um Dampfbildung beim Heißreinigen des Dialysierflüssigkeitssystems zu verhindern, weil sich bei Dampfbildung die Strömungseigenschaften im Dialysierflüssigkeitssystem ändern und der Dampf aus dem Dialysierflüssigkeitssystem austreten und in das Gehäuseinnere der Blutbehandlungs-Vorrichtung gelangen kann, wo der Dampf kondensieren und zu Schäden führen kann.

Die Siedetemperatur sinkt mit abnehmendem Druck. Die Änderung der Siedetemperatur mit dem Absolutdruck ist qualitativ bekannt und kann aus der Fachliteratur, beispielsweise aus Tabellenwerken für Wasser entnommen und in der zentralen Steuerung der Blutbehandlungs-Vorrichtung hinterlegt werden.

Bei der Heißdesinfektion des Dialyatsystems muss das gesamte Dialysierflüssigkeitssystem bei einem bekannten Gerät auf mehr als 80 °C erhitzt werden. Besonders vorteilhaft wird das gesamte Dialysierflüssigkeitssystem durch Spülen mit heißem Reinwasser von rund 85 °C gereinigt. Bei der Heißreinigung wird das gesamte Dialysierflüssigkeitssystem mit einem vorgegebenen Fluss erhitzten Reinwassers gespült. Das Reinwasser wird mit einem elektrischen Heizstab erhitzt. Um die Aufheizdauer des Reinwassers möglichst kurz zu halten, soll die Reinwassertemperatur am Heizstab möglichst hoch sein. Die Reinwassertemperatur am Heizstab im Dialysierflüssigkeitssystem wird stets unter der Siedetemperatur geregelt.

Der Absolutdruck kann für die Bestimmung weiterer Betriebsparameter sinnvoll sein.

Für die Ermittlung und Bereitstellung mancher Betriebsparameter wäre allerdings das Vorliegen eines aktuellen Messwerts des absoluten Umgebungsdrucks, auch einfach als Absolutdruck bezeichnet, vorteilhaft. Bei Vorliegen eines Messwerts des Absolutdrucks könnten solche Betriebsparameter mit besonders hoher Genauigkeit ermittelt und eingestellt werden.

Eine Aufgabe der Erfindung ist es, ein gattungsgemäßes Blutbehandlungsgerät ohne eigenen Absolutdruckmesser derart weiterzubilden, dass das Einstellen mindestens eines vom Absolutdruck abhängigen Betriebsparameters automatisch und ohne einen Absolutdrucksensor erfolgen kann und ohne dass der Einsatz eines qualifizierten Servicetechnikers oder eines Benutzers erforderlich ist.

Eine weitere Aufgabe der vorliegenden Erfindung ist es, mindestens einen vom Absolutdruck abhängigen Betriebsparameter mit hoher Genauigkeit zu ermitteln, ohne dass dafür ein Absolutdruckmesser erforderlich ist.

Eine weitere Aufgabe der vorliegenden Erfindung ist es, mindestens einen vom Absolutdruck abhängigen Betriebsparameter automatisch anzupassen, wenn sich der Absolutdruck in der Umgebung wesentlich ändert.

Eine weitere Aufgabe der vorliegenden Erfindung ist die Verbesserung der Benutzerfreundlichkeit, so dass der Anwender, beispielsweise der behandelnde Arzt oder die Dialyseschwester, nicht mit der Einstellung der vom Absolutdruck abhängigen Betriebsparameter belastet werden und dafür auch keine Ausfallzeiten der Blutbehandlungsmaschine für den Einsatz eines qualifizierten Servicetechniker entstehen.

Eine weitere Aufgabe der vorliegenden Erfindung ist die Erhöhung der Zuverlässigkeit der Blutbehandlungsvorrichtung. Je genauer die vom Absolutdruck abhängigen Betriebsparameter eingestellt werden können, desto zuverlässiger kann das Dialysierflüssigkeitssystem und damit die Blutbehandlungsvorrichtung arbeiten.

Eine weitere Aufgabe der vorliegenden Erfindung ist die Erhöhung der Sicherheit der Blutbehandlungsvorrichtung. Je genauer die vom Absolutdruck abhängigen Betriebsparameter eingestellt werden können, desto sicherer kann das Dialysierflüssigkeitssystem arbeiten.

Die Lösung dieser Aufgaben erfolgt erfindungsgemäß mit den Merkmalen der unabhängigen Patentansprüche 1, 7, 14 und 18. Vorteilhafte Ausführungsformen sind Gegenstand der Unteransprüche.

Nach der Lehre der Erfindung werden diese Aufgaben dadurch gelöst, dass in einen geschlossenen zumindest teilweise mit Luft gefüllten Behälter mit im wesentlichen konstantem Behältervolumen durch Druckausgleich gegenüber der Umgebung zunächst der absolute Umgebungsdruck eingestellt und durch Absperren des Behälters gegenüber der Umgebung konstant gehalten wird und danach mit einem Fördermittel eine vorgegebene Abfolge von mindestens zwei Förderhüben einer Flüssigkeit in dem Behälter gefördert wird und nach jedem Förderhub der gestiegene Relativdruck in dem Behälter gemessen wird und nach jedem Förderhub das insgesamt geförderte Volumen und der Relativdruck einem Wertepaar zugewiesen werden und der Absolutdruck und das initiale Luftvolumen unter Verwendung der Wertepaare für die mindestens zwei erfolgte Förderhübe berechnet wird. Die Berechnung kann auf der Basis des Gesetzes von Boyle-Mariotte erfolgen. Nach der Lehre der Erfindung werden die Aufgaben weiter dadurch gelöst, dass in einem weiteren Schritt die vom Absolutdruck abhängigen Betriebsparameter berechnet werden.

Das Einstellen des mindestens einen Betriebsparameters kann in manchen Ausführungsformen ein Berechnen und/oder Speichern in einem Datenspeicher in einer Rechen- und Steuereinheit umfassen. In manchen Ausführungsformen kann das Einstellen des mindestens einen Betriebsparameters ein Ausführen eines Stelleingriffs an einer Funktionseinheit der Vorrichtung zur extrakorporalen Blutbehandlung umfassen. Ein Beispiel für eine Funktionseinheit ist eine Pumpe mit einem ansteuerbaren Pumpenantrieb.

Alle mit dem erfindungsgemäßen Verfahren erzielbaren Vorteile lassen sich in bestimmten erfindungsgemäßen Ausführungsformen ungeschmälert auch mit der erfindungsgemäßen Vorrichtung und/oder mit der Vorrichtung zur extrakorporalen Blutbehandlung erzielen. Die gilt in einigen erfindungsgemäßen Ausführungsformen auch für das erfindungsgemäße Computer-Programm-Produkt.

Das erfindungsgemäße Verfahren kann automatisch ablaufen, ohne dass ein Benutzereingriff erforderlich ist. Das erfindungsgemäße Verfahren kann von einer Steuer- und Recheneinheit, die Teil der erfindungsgemäßen Vorrichtung sein kann, ausgeführt werden. Das Starten des erfindungsgemäßen Verfahrens kann beispielsweise in regelmäßigen Zeitintervallen automatisch von der Steuer- und Recheneinheit durchgeführt werden. Auf dem Display der Blutbehandlungsvorrichtung kann während der Durchführung eine Meldung angezeigt werden, dass eine automatische Aktualisierung der vom Absolutdruck abhängigen Betriebsparameter erfolgt. Das Starten der Blutbehandlung kann während der Aktualisierung von der Steuer- und Recheneinheit unterbunden werden.

In der Steuer- und Recheneinheit kann eine Begrenzung des Relativdrucks auf einen Maximalwert vorgesehen sein, so dass keine unzulässig hohen Drücke auftreten können. Es kann eine Fehlermeldung auf dem Display der Blutbehandlungsvorrichtung erfolgen, die auf ein Fehlschlagen der Aktualisierung der vom Absolutdruck abhängigen Betriebsparameter hinweist. Beim Erreichen eines unzulässig hohen Relativdrucks wird das erfindungsgemäße Verfahren abgebrochen und kann zu einem späteren Zeitpunkt erneut gestartet werden.

Nach der Lehre der Erfindung werden die Aufgaben weiter dadurch gelöst, dass der mindestens eine berechnete Betriebsparameter an der Blutbehandlungsmaschine eingestellt wird.

Allein auf der Grundlage der Messwerte des relativen Drucks in dem geschlossenen Behälter wird der Absolutdruck berechnet. Der mindestens eine vom Absolutdruck abhängige Betriebsparameter kann von der zentralen Steuer- und Recheneinheit der Blutbehandlungs-Vorrichtung berechnet und in einem Datenspeicher gespeichert und/oder durch Steuer- oder Regeleingriffe eingestellt werden. Der Einsatz eines Absolutdruckmessers ist dafür nicht erforderlich.

Die erfindungsgemäße Vorrichtung sieht einen geschlossenen Behälter mit im Wesentlichen konstantem Innenvolumen vor. Unter einem im Wesentlichen konstanten Behältervolumen ist zu verstehen, dass sich das Innenvolumen mit steigendem Innendruck in dem relevanten Druckbereich nicht oder nur vernachlässigbar ändert. Unter geschlossen ist zu verstehen, dass während der Durchführung des erfindungsgemäßen Verfahrens keine freie Öffnung zur Umgebung besteht und in den Behälter mündende, nicht für die Durchführung des Verfahrens benötigten Zuleitungen oder Ableitungen abgesperrt sind. Zuleitungen oder Ableitungen können beispielsweise Schläuche oder Rohrleitungen umfassen. Die Zuleitungen oder Ableitungen können durch Ventile verschlossen sein. Die Zuleitungen oder Ableitungen können durch abgeschaltete Pumpen verschlossen sein. Die nicht für die Durchführung des erfindungsgemäßen Verfahrens benötigten Zuleitungen oder Ableitungen werden vor der Durchführung des erfindungsgemäßen Verfahrens von der Steuer- und Recheneinheit durch Stelleingriffe verschlossen.

In einer bevorzugten Ausführungsform handelt es sich bei dem Behälter um eine Mischkammer in einem Dialysierflüssigkeitssystem einer Blutbehandlungsvorrichtung zur Herstellung von frischer Dialysierflüssigkeit.

Die erfindungsgemäße Vorrichtung weist Mittel zum Messen des Relativdrucks in dem Behälter auf. Bei den Mitteln zum Messen des Relativdrucks kann es sich um einen Drucksensor in dem Behälter handeln. Insbesondere kann es sich um einen Drucksensor in dem Luftvolumen im Behälterinneren handeln. Es kann sich aber auch um einen Drucksensor handeln, der mit dem Behälterinneren zum Messen des Drucks in funktioneller Verbindung steht. Der Drucksensor gibt in Abhängigkeit vom Relativdruck im Behälter ein elektrisches Drucksignal ab, das über eine Datenleitung an die Steuer- und Recheneinheit weitergeleitet wird. Es ist aber auch möglich, dass der Drucksensor das Drucksignal drahtlos an die Steuer- und Recheneinheit übermittelt. Es kann sich bei dem Drucksensor um einen RFID-Sensor handeln. Es kann sich bei den Mitteln zum Messen des Relativdrucks um einen in der Mischkammer eines Dialysierflüssigkeitssystems ohnehin vorhandenen Relativdrucksensor handeln.

Die erfindungsgemäße Vorrichtung sieht Mittel zum Einstellen des Absolutdrucks (absoluten Umgebungsdrucks) in dem Behälter vor. Der Betrag des Absolutdrucks ist dabei zunächst noch unbekannt und gesucht. Bei den Mitteln zum initialen Einstellen des Absolutdrucks kann es sich um ein Absperrventil handeln, mit dem eine Öffnung des ansonsten geschlossenen Behälters gegenüber der Umgebung geöffnet oder für die Durchführung der weiteren Schritte des erfindungsgemäßen Verfahrens abgesperrt werden kann. Die Mischkammer kann als Öffnung eine Leitung zur Umgebung aufweisen, deren Ende gegen die Umgebung offen ist. In der Leitung ist ein Absperrventil angeordnet, mit dem die Leitung luftdicht verschließbar ist. Der Umgebungsdruck kann in dem Behälter auf einfache Weise durch kurzzeitiges Öffnen des Absperrventils gegenüber der Umgebung eingestellt werden, wobei ein Druckausgleich gegenüber der Umgebung erfolgt und sich der absolute Umgebungsdruck selbsttätig in dem Behälter einstellt. Nach dem Druckausgleich wird das Absperrventil geschlossen. Der eingestellte Druck bleibt beim Schließen des Absperrventils in dem Behälter erhalten. Das Absperrventil bleibt während der nachfolgenden Schritte des erfindungsgemäßen Verfahrens stets gegenüber der Umgebung geschlossen, so dass kein erneuter Druckausgleich gegenüber der Umgebung erfolgen kann. Erst nach der vollständigen Durchführung des erfindungsgemäßen Verfahrens wird ein erneuter Druckausgleich gegenüber der Umgebung veranlasst, um den aufgebauten Überdruck zu entspannen.

Das Ventil kann automatisch betätigbar sein oder betätigt werden. Das Ventil kann von der Steuer- und Recheneinheit der erfindungsgemäßen Vorrichtung geöffnet und geschlossen werden. Das Ventil kann aber auch durch einen manuellen Stelleingriff des Benutzers betätigt werden. Es kann sich beispielsweise um ein Magnetventil handeln, das elektrisch ansteuerbar ist. Es kann sich aber auch um ein pneumatisch ansteuerbares Ventil handeln.

Nach dem Einstellen des Umgebungsdrucks liegt in dem Behälter ein Luftvolumen vor, das nachfolgend als initiales Luftvolumen bezeichnet wird. Das initiale Luftvolumen füllt den Behälter zumindest teilweise. Das restliche Behältervolumen kann mit einem initialen Flüssigkeitsvolumen gefüllt sein, das einen Flüssigkeitsspiegel ausbildet. Es ist auch möglich, dass das komplette Innenvolumen des Behälters mit Luft gefüllt ist und das initiale Luftvolumen bildet. Es befindet sich dann keine Flüssigkeit in dem Behälter.

In dem Behälter können Mittel zum Messen des Flüssigkeitsvolumens vorhanden sein. Bei den Mitteln zum Messen des Flüssigkeitsvolumens kann es sich um eine Füllstandsmessung mittels eines Füllstandssensors handeln. Der Füllstandssensor gibt in Abhängigkeit vom Füllstand im Behälter ein elektrisches Füllstandssignal ab, das über eine Datenleitung zu der Steuer- und Recheneinheit weitergeleitet wird. Das Flüssigkeitsvolumen kann aus dem gemessenen Füllstand und der bekannten Geometrie des Behälters von der Steuer- und Recheneinheit berechnet werden. Das initale Luftvolumen kann als Differenz aus dem Behältervolumen und dem initalen Flüssigkeitsvolumen berechnet werden. Für die Durchführung des erfindungsgemäßen Verfahrens sind Mittel zum Messen des Flüssigkeitsvolumens zwar nicht erforderlich, weil das initiale Luftvolumen mit dem erfindungsgemäßen Verfahren berechnet werden kann. In der Mischkammer eines Dialysierflüssigkeitssystems kann eine Füllstandsmessung jedoch ohnehin vorhanden sein, so dass diese vorteilhaft zur Plausibilitätskontrolle des Berechnungsergebnisses für das initiale Luftvolumen verwendet werden kann. Es ist natürlich auch möglich, dass initiale Luftvolumen nur mittels einer Füllstandmessung zu ermitteln.

Auch ist es möglich, mittels einer Füllstandmessung in dem Behälter bei bekannten Anfangsbedingungen des Drucks und des Luftvolumens in dem Behälter als Alternative zum erfindungsgemäßen Verfahren eine gemessene Füllstandsänderung als Maß für eine Druckänderung in dem Behälter zu auszuwerten und daraus auf den Absolutdruck zu schließen.

In den Behälter mündet mindestens eine mit einer Flüssigkeitsquelle in fluidführender Verbindung stehende Flüssigkeitsleitung, in der mindestens ein Fördermittel zum Fördern der Flüssigkeit in einer ersten Förderrichtung in den Behälter hinein angeordnet ist. Das Fördermittel zeichnet sich dadurch aus, das beim Stillstand des Fördermittels keine Rückströmung durch das Fördermittel in entgegen gesetzter Richtung zur ersten Förderrichtung erfolgt. Das Fördermittel wirkt wie ein Rückschlagventil.

Die Förderleistung des Fördermittels, insbesondere die Fördermenge des Fördermittels pro Förderhub und/oder pro Zeiteinheit ist bekannt und in der Steuer- und Recheneinheit hinterlegt. In einer bevorzugten Ausführungsform kann es sich bei dem Förderhub um einen Pumpenhub handeln. Die Flüssigkeit ist inkompressibel oder wird als inkompressibel angenommen.

Das mindestens eine Fördermittel kann über die Steuer- und Recheneinheit zum Starten und Stoppen und/oder zum Ausführen mindestens eines Förderhubs angesteuert werden. Das mindestens eine Fördermittel kann über die Steuer- und Recheneinheit zum Ausführen einer Abfolge von Förderhüben angesteuert werden. Die Förderhübe können das gleiche Hubvolumen aufweisen. Es ist aber für die Ausführung der Erfindung nicht erforderlich, dass alle Förderhübe das gleiche Hubvolumen aufweisen. In einer bevorzugten Ausführungsform weisen alle Förderhübe das gleiche Hubvolumen auf.

In einer bevorzugten Ausführungsform ist die Flüssigkeitsquelle eine aus der Gruppe Zuführung von Reinwasser, Zuführung von Natriumbicarbonatkonzentrat und Zuführung von Säurekonzentrat aus dem Dialysierflüssigkeitssystem einer Blutbehandlungsvorrichtung.

In einer bevorzugten Ausführungsform handelt es sich bei dem Förderhub um den Pumpenhub einer Membranpumpe. Das Förderverhalten einer Membranpumpe ist von Förderhub zu Förderhub diskontinuierlich. Das Fördervolumen eines vollständigen Pumpenhubs einer Membranpumpe ist allein von der Geometrie der Membranpumpe abhängig. Mit jedem vollständigen Pumpenhub kann stets das gleiche Flüssigkeitsvolumen gefördert werden.

In einer weiteren bevorzugten Ausführungsform handelt es sich um den Pumpenhub einer Kolbenpumpe. Das Förderverhalten einer Kolbenpumpe ist von Förderhub zu Förderhub diskontinuierlich. Der Pumpenhub einer Kolbenpumpe ist allein von der Geometrie der Kolbenpumpe abhängig. Mit jedem vollständigen Pumpenhub kann stets das gleiche Flüssigkeitsvolumen gefördert werden.

In einer weiteren bevorzugten Ausführungsform handelt es sich bei dem Pumpenhub um eine vorgegebene Förderdauer eines Fördermittels mit kontinuierlichem Förderverhalten. Bei dem Fördermittel kann es sich beispielsweise um eine Zahnradpumpe oder um eine Schlauchpumpe handeln.

Bei dem Fördermittel kann es sich in einer bevorzugten Ausführungsform um eine Dosierpumpe handeln. Die Dosierpumpe kann hochgenau sein. Die Dosierpumpe kann eine Membranpumpe sein.

In einer besonders bevorzugten Ausführungsform kann der Pumpenhub durch Vorgabe einer Schrittzahl oder eines Schrittwinkels an einen Schrittmotor, der das Fördermittel antreibt, vorgegeben werden. Der Schrittmotor kann elektrisch betrieben werden. Der Schrittwinkel und/oder die Schrittzahl können durch elektrische Impulse einer Steuer- und Recheneinheit vorgegeben werden.

Eine Änderung des Drucks in dem Behälter nach dem Einstellen des Umgebungsdrucks soll bei dem erfindungsgemäßen Verfahren ausschließlich durch die Zufuhr von Flüssigkeit durch die Pumpenhübe des Fördermittels erfolgen.

Die Ansteuerung des Fördermittels kann durch eine Steuer- und Recheneinheit erfolgen. Die Steuer- und Recheneinheit kann konfiguriert sein, um eine Abfolge von Förderhüben des Fördermittels zu veranlassen. Die Abfolge von Förderhüben weist eine vorgegebene Anzahl an Förderhüben auf.

Die Steuer- und Recheneinheit kann konfiguriert sein, um nach jedem erfolgten Förderhub einen Druckmesswert in dem Behälter zu erfassen und/oder zu speichern.

Die Steuer- und Recheneinheit kann zusätzlich konfiguriert sein, um nach jedem erfolgten Förderhub das insgesamt geförderte Flüssigkeitsvolumen zu berechnen. Unter dem insgesamt geförderten Flüssigkeitsvolumen ist die Summe der Flüssigkeitsvolumina aller bereits während der Durchführung des erfindungsgemäßen Verfahrens erfolgten Förderhübe zu verstehen.

In einer bevorzugen Ausführungsform weist das Volumen jedes Förderhubs stets denselben Betrag, nämlich ein konstantes Hubvolumen auf. In dieser Ausführungsform wird das insgesamt geförderte Flüssigkeitsvolumen auf besonders einfache Weise als Produkt aus dem Betrag des Hubvolumens und der Anzahl der erfolgten Förderhübe berechnet.

Die Steuer- und Recheneinheit kann zusätzlich konfiguriert sein, um nach jedem erfolgten Förderhub die Beträge des insgesamt geförderten Flüssigkeitsvolumens und des Relativdrucks in dem Behälter einem Wertepaar zuzuweisen und zu speichern.

Die Steuer- und Recheneinheit kann zusätzlich konfiguriert sein, um nach Beendigung und Auswertung des letzten Förderhubs einer vorgegebenen Abfolge von Förderhüben automatisch als nächsten Schritt die Berechnung des Absolutdrucks und des initialen Luftvolumens auf der Basis aller Wertepaare zu starten.

Die Steuer- und Recheneinheit kann zusätzlich konfiguriert sein, um auf der Basis aller Wertepaare mit dem folgenden Ansatz, der durch Anwendung des Ge-setzes von Boyle-Mariotte auf die Zustandsänderungen in dem Behälter erhalten wird, auf dem Wege einer Ausgleichsrechnung die gesuchten Parameter Absolutdruck und initiales Luftvolumen derart zu berechnen, dass eine bestmögliche Annäherung der durch den Ansatz beschriebenen Funktion an die Messwerte des Relativdrucks erhalten wird:

$$P_i = P_{abs,amb} \cdot \frac{i \cdot V_{pump}}{V_{vessel,air,0} - i \cdot V_{pump}}; \ i = 1,...,n$$

In der Gleichung (1) bedeutet $P_{abs,amb}$ den gesuchten Absolutdruck in der Umgebung, $V_{vessel,air,0}$ das gesuchte initiale Luftvolumen in der Mischkammer, $V_{pump}$ das Hubvolumen der hier verwendeten Membranpumpe, $n$ die gesamte Anzahl der Förderhübe der Membranpumpe, $i$ den Laufindex für die Anzahl der Pumpenhübe und $P$ den Relativdruck in der Mischkammer nach Durchführung eines Pumpenhubs.

Die Steuer- und Recheneinheit kann zusätzlich konfiguriert sein, um in einem weiteren Schritt den mindestens einen vom Absolutdruck abhängigen Betriebsparameter zu berechnen.

Die Steuer- und Recheneinheit kann zusätzlich konfiguriert sein, um mittels Stelleingriffen den mindestens einen vom Absolutdruck abhängigen, berechneten Betriebsparameter in der Blutbehandlungsvorrichtung einzustellen. Das Einstellen des mindestens einen Betriebsparameters kann durch Steuereingriffe und/oder Regeleingriffe einer Steuer- und Recheneinheit erfolgen.

Die Steuer- und Recheneinheit kann zusätzlich konfiguriert sein, die Beheizung des Reinwassers, die mittels einer elektrischen Heizung erfolgen kann, so zu regeln, dass die Temperatur des Reinwassers stets unter einem Temperaturgrenzwert gehalten wird, der unter der Siedetemperatur liegt. Dazu kann die Steuer- und Recheneinheit die Heizleistung und/oder die Einschaltdauer der elektrischen Heizung regeln. Bei der elektrischen Heizung kann es sich um einen Heizstab handeln.

Die Steuer- und Recheneinheit kann zusätzlich konfiguriert sein, um den gewünschten Entgasungsdruck als Unterdruck in Bezug auf den Absolutdruck durch einen Stelleingriff an der Entgasungspumpe des Dialysierflüssigkeitssystems einzustellen. Der Entgasungsdruck kann mittels der drehzahlgeregelten Entgasungspumpe als Druckabfall an einer Entgasungsdrossel in der Reinwasserleitung eingestellt werden. Bei der Entgasungsdrossel kann es sich um ein Drosselventil handeln.

Die erfindungsgemäße Vorrichtung kann eine selbständige Einheit bilden, aber auch Bestandteil einer Blutbehandlungsvorrichtung sein. Es kann sich um eine Vorrichtung zum Nachrüsten einer Blutbehandlungsvorrichtung handeln, die keinen eigenen erfindungsgemäßen Behälter aufweist.

Eine besonders bevorzugte Ausführungsform sieht vor, dass die erfindungsgemäße Vorrichtung Bestandteil der Blutbehandlungsvorrichtung ist, weil die bekannten Blutbehandlungsvorrichtungen bereits über Komponenten verfügen, von denen die erfindungsgemäße Vorrichtung Gebrauch machen kann.

In einer weiteren besonders bevorzugten Ausführungsform kann sich die Ausrüstung einer Blutbehandlungsvorrichtung mit der erfindungsgemäßen Vorrichtung auf eine Aktualisierung der Software der Steuer- und Recheneinheit beschränken, weil über die Software hinaus bereits alle benötigten Komponenten in der Blutbehandlungsvorrichtung vorhanden sind. Für solche Ausführungsformen und andere Ausführungsformen wird ein erfindungsgemäßes Computer-Programm-Produkt mit einem auf einem maschinenlesbaren Träger gespeicherten Programmcode bereitgestellt.

Die erfindungsgemäße Vorrichtung und das erfindungsgemäße Verfahren können bei den bekannten Blutbehandlungsvorrichtungen eingesetzt werden, ohne dass hierzu größere Umbaumaßnahmen erforderlich sind. Eine Mischkammer in einem Dialysierflüssigkeitssystem ist bei den bekannten Blutbehandlungsvorrichtungen ohnehin vorhanden. Auch die Mittel zum Messen des Relativdrucks sind an den bekannten Blutbehandlungsvorrichtungen ohnehin vorhanden. An Blutbehandlungsvorrichtungen, die nicht über eine Mischkammer im Dialysierflüssigkeitssystem verfügen, ist eine Nachrüstung einer geeigneten Kammer ohne großen Aufwand möglich. Geeignete Fördermittel zum Fördern einer Flüssigkeit sind im Dialysierflüssigkeitssystem der bekannten Blutbehandlungsvorrichtungen stets vorhanden.

Im Folgenden wird ein Ausführungsbeispiel der Erfindung unter Bezugnahme auf die Figuren näher erläutert. Anhand des in den Figuren dargestellten Ausführungsbeispiels werden weitere Einzelheiten und Vorteile der Erfindung näher beschrieben. Das erfindungsgemäße Verfahren und die erfindungsgemäße Vorrichtung werden am Beispiel einer als Hämodialyse-Vorrichtung ausgestalteten Blutbehandlungsvorrichtung beschrieben. Das erfindungsgemäße Verfahren kann in gleicher Weise aber auch bei anderen Blutbehandlungsvorrichtungen, beispielsweise einer Hämodiafiltrationsvorrichtung Verwendung finden.

Es zeigen:

Figur 1     ein Flussbild des Dialyatsystems einer einer als Hämodialysevorrichtung ausgestalteten Blutbehandlungsvorrichtung mit einer Mischkammer;

Figur 2     eine grafische Darstellung der Druckverhältnisse des Relativdrucks in der Mischkammer des Dialysierflüssigkeitssystems aus Figur 1 bei der Durchführung des erfindungsgemäßen Verfahrens.

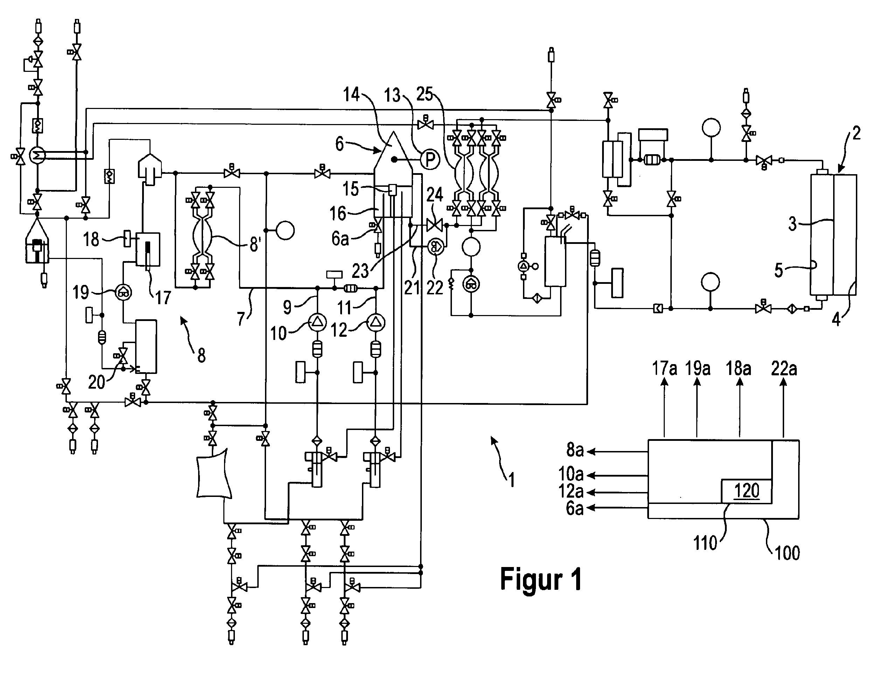

Figur 1 zeigt in vereinfachter schematischer Darstellung die wesentlichen Komponenten des Dialysierflüssigkeitssystems 1 einer als Hämodialysevorrichtung ausgestalteten Blutbehandlungsvorrichtung. Bei dem vorliegenden Ausführungsbeispiel wird die als Hämodialyse-Vorrichtung ausgestaltete Blutbehandlungsvorrichtung im Weiteren vereinfacht als "Blutbehandlungsvorrichtung" bezeichnet, die einen Dialysator 2 aufweist, der schematisch durch eine semipermeable Membran 3 in eine Blutkammer 4 und eine Dialysierflüssigkeitskammer 5 getrennt ist. Die Blutkammer ist Teil des extrakorporalen Blutkreislauf (nicht gezeigt) und die Dialysierflüssigkeitskammer 5 ist Teil des Dialysierflüssigkeitssystems 1. Die zentrale Rechen- und Steuereinheit 100 betreibt und überwacht die Blutbehandlungsvorrichtung und das Dialysierflüssigkeitssystem. Die Rechen- und Steuereinheit 110 der erfindungsgemäßen Vorrichtung ist im Ausführungsbeispiel Teil der zentralen Rechen- und Steuereinheit 100 der Blutbehandlungsvorrichtung. Die Rechen- und Steuereinheit 110 kann aber auch getrennt von der zentralen Rechen- und Steuereinheit 100 sein und mit dieser über Datenleitungen verbunden sein.

Das Dialysierflüssigkeitssystem verfügt über eine Mischkammer 6 zum Anmischen frischer Dialysierflüssigkeit aus Reinwasser und Flüssigkonzentraten. Das Dialysierflüssigkeitssystem verfügt über eine Leitung 7 zum Führen von Reinwasser, in die eine passive Membrankammer 8ᴵ geschaltet ist, die erst im Zusammenwirken mit einer stromaufwärts angeordneten Zahnradpumpe 19 (Entgasungspumpe) ein Fördermittel mit Dosierfunktion bildet, dass nachfolgend zusammengefasst als Fördermittel 8 bezeichnet wird. Die 7 Leitung mündet in die Mischkammer 6. Bei dem Fördermittel 8 handelt es sich um ein Fördermittel für Reinwasser.

Eine Leitung 9 mündet stromabwärts vom Fördermittel für Reinwasser 8 in die Leitung 7. In die Leitung 9 ist ein Fördermittel 10 geschaltet. Bei dem Fördermittel 10 handelt es sich in dem Ausführungsbeispiel um eine Dosierpumpe für Natriumbicarbonat-Konzentrat. Die Dosierpumpe 10 ist als Membranpumpe ausgeführt.

Eine weitere Leitung 11 mündet ebenfalls stromabwärts vom Fördermittel für Reinwasser 8 in die erste Leitung 7. In die Leitung 11 ist ein Fördermittel 12 geschaltet. Bei dem Fördermittel 11 handelt es sich in dem Ausführungsbeispiel um eine Dosierpumpe für Säure-Konzentrat. Die Dosierpumpe 12 ist als Membranpumpe ausgeführt.

Eine Leitung 21 führt stromabwärts von der Mischkammer 6 zur Dialysierflüssigkeitskammer 5 des Dialysators 2. In die Leitung 21 ist ein Fördermittel 22 geschaltet. Bei dem Fördermittel handelt es sich in dem Ausführungsbeispiel um eine Zahnradpumpe 22, die Teil einer Bilanziervorrichtung 25 ist. In Parallelschaltung zur Leitung 21 befindet sich eine Bypass-Leitung 23 mit einem Bypass-Ventil 24. Das Bypass-Ventil 24 ist während des Betriebs der Zahnradpumpe 22 geschlossen.

Prinzipiell kann im Ausführungsbeispiel für die Durchführung des erfindungsgemäßen Verfahrens jedes der Fördermittel 8, 10, 12 oder 22 zum Hineinfördern einer Flüssigkeit in den Behälter 6 oder zum Herausfördern aus dem Behälter 6 ausgewählt werden. Zum Hineinfördern in den Behälter 6 müssten die Fördermittel 8, 10, und 12 in normaler Förderrichtung betrieben werden während das Fördermittel 22 in umgekehrter Förderrichtung betrieben werden müsste. Zum Herausfördern aus dem Behälter 6 müssten die Fördermittel 8, 10, und 12 in umgekehrter Förderrichtung betrieben werden, während das Fördermittel 22 in normaler Förderrichtung betrieben werden müsste. Im vorliegenden Ausführungsbeispiel wird die Durchführung des erfindungsgemäßen Verfahrens beispielhaft mit dem Fördermittel 10 erläutert, mit dem Flüssigkeit in normaler Förderrichtung in den Behälter 6 gefördert wird.

Die Steuer- und Recheneinheit 110 kann Mittel zum Auswählen eines der Fördermittel (8, 10, 12, 22) zum Durchführen des erfindungsgemäßen Verfahrens aufweisen. Die Auswahl kann in der Steuer- und Recheneinheit 110 auch fest eingestellt sein oder durch den Anwender durch Benutzereingriff, beispielsweise über den Touchscreen der Blutbehandlungsvorrichtung (in Figur 1 nicht gezeigt), eingestellt werden.

In dem Ausführungsbeispiel wird die Membranpumpe 10 für Flüssigkonzentrat von der Steuer- und Recheneinheit 110 als Fördermittel ausgewählt. Ein Förderhub der Membranpumpe 10 entspricht im vorliegenden Beispiel genau dem Hubvolumen, das bei einem vollständigen Pumpenhub gefördert wird. Dieses Hubvolumen eines vollständigen Pumpenhubs ist für die ausgewählte Pumpe bekannt und konstant. Der Förderhub könnte alternativ aber auch einen Teil eines vollständigen Pumpenhubs umfassen, beispielsweise wenn der Pumpenantrieb ein Schrittmotor ist.

Die Steuer- und Recheneinheit 110 weist Mittel zum Veranlassen einer vorgegebenen Anzahl von Förderhüben auf. Die Förderhübe werden durch Steuereingriffe 10a veranlasst.

Die erfindungsgemäße Vorrichtung weist einen Drucksensor 13 auf, der den relativen Druck in der Mischkammer 6 misst. Die Messwerte des Drucksensors 13 werden an die Steuer- und Recheneinheit 110 übertragen und liegen dort in dem Datenspeicher 120 zur Auswertung vor. Des Weiteren weist die Steuer- und Recheneinheit 110 Mittel zum Zuordnen des nach jedem Förderhub geförderten Flüssigkeitsvolumens zu dem gemessenen Relativdruck in der Mischkammer als Wertepaar für den erfolgten Förderhub auf. Die Wertepaare aller Förderhübe werden in der Steuer- und Recheneinheit 110 gespeichert.

Des Weiteren weist die Steuer- und Recheneinheit 110 Mittel zum Berechnen des absoluten Umgebungsdrucks und des initialen Luftvolumens unter Verwendung der gespeicherten Wertepaare für alle Förderhübe auf.

Die Berechnungen der gesuchten Parameter erfolgen mit Hilfe eines Computerprogramms mit Programmcode zur Veranlassung der maschinellen Schritte des Verfahrens und zur Auswertung des Messergebnisse. Die Berechnungsgleichungen sind in dem Programmcode implementiert. Der Programmcode ist in der Steuer- und Recheneinheit 110 gespeichert.

Das Computerprogramm liegt als Computer-Programm-Produkt mit dem auf einem maschinenlesbaren Träger gespeicherten Programmcode zur Veranlassung der maschinellen Schritte des Verfahrens vor. Das Computerprogramm läuft in der Steuer- und Recheneinheit 110 ab. Die Steuer- und Recheneinheit 110 verfügt über einen Datenspeicher 120.

Das Computerprogramm mit Programmcode zur Veranlassung der maschinellen Schritte des Verfahrens und zur Auswertung der Messergebnisse startet die Berechnungen, sobald die gesamte vorgegebene Anzahl an Förderhüben abgeschlossen ist.

Das gesamte Innenvolumen der Mischkammer 6 ist bekannt und beträgt in dem Ausführungsbeispiel 350 ml. Das initiale Flüssigkeitsvolumen 16 in der Mischkammer 6 wird aus dem Messwert der Füllstandsmessvorrichtung 15 berechnet. Das initiale Luftvolumen 14 in der Mischkammer wird von der Steuer- und Recheneinheit 110 als Differenz des gesamten Innenvolumens und des Flüssigkeitsvolumens berechnet und beträgt in dem Ausführungsbeispiel 242 ml. Die Temperatur in der Mischkammer beträgt 37 °C und wird während der Durchführung des erfindungsgemäßen Verfahrens als konstant angenommen.

Die Anzahl der durchzuführenden Förderhübe beträgt vorteilhaft über 50 und besonders vorteilhaft bis zu 120. Mehr als 120 Förderhübe sind in dem Ausführungsbeispiel allerdings nicht erforderlich und führen nicht zu einem genaueren Ergebnis. Die Anzahl n der durchzuführenden Förderhübe ist in dem Ausführungsbeispiel beispielhaft mit n = 110 in der zentralen Steuerung der Blutbehandlungsvorrichtung vorgegeben. Das Hubvolumen des der Membranpumpe 10 beträgt in dem Ausführungsbeispiel 1,0 ml (ein Milliliter) und entspricht dem Fördervolumen eines einzelnen Förderhubs der Membranpumpe 10. Die Steuer- und Recheneinheit 110 startet und stoppt die Förderhübe der Membranpumpe durch Steuereingriffe 10a. Es wird mit jedem Förderhub ein Milliliter Flüssigkeit in die Mischkammer 6 gepumpt. Eine Rückströmung der Flüssigkeit durch die Membranpumpe 10 findet nicht statt. Der Druck in der Mischkammer erhöht sich mit jedem Förderhub entsprechend dem Gesetz von Boyle-Mariotte, weil das Flüssigkeitsvolumen mit jedem Förderhub um den Betrag eines Förderhubs zunimmt und im Gegenzug das Luftvolumen mit jedem Förderhub in gleichem Maße um den Betrag des Förderhubs abnimmt. Die Luft wird deshalb mit jedem Förderhub um denselben Betrag komprimiert. Dieser Zusammenhang wird durch die Gleichung (1) beschrieben. Die thermodynamische Grundlage der Gleichung (1) ist das Gesetzes von Boyle-Mariotte, angewendet auf die durch die Förderhübe verursachten Zustandsänderungen des Luftvolumens in dem Mischbehälter:

$$P_{abs,amb} \cdot V_{vessel,air,0} = (P_{abs,amb} + P_i) \cdot (V_{vessel,air,0} - i \cdot V_{pump}); \; i = 1, ..., n \; \textbf{(1)}$$

In der Gleichung (1) bedeutet $P_{abs,amb}$ den gesuchten Absolutdruck in der Umgebung, $V_{vessel,air,0}$ das gesuchte initiale Luftvolumen in der Mischkammer, $V_{pump}$ das Hubvolumen der Membranpumpe, n die gesamte Anzahl der Förderhübe der Membranpumpe, i den Laufindex für die Anzahl der Pumpenhübe und $P_i$ den Relativdruck in der Mischkammer nach dem i-ten Pumpenhub.

Die Gleichung (2) wird durch Umformung aus der Gleichung (1) erhalten und in dem Programmcode für eine Ausgleichsrechnung benutzt, um die gesuchten Parameter $P_{abs,amb}$ und $V_{vessel,air,0}$ zu berechnen, wobei kein Füllstandssensor benötigt wird:

$$P_i = P_{abs,amb} \cdot \frac{i \cdot V_{pump}}{V_{vessel,air,0} - i \cdot V_{pump}}; \; i = 1, ..., n \; \textbf{(2)}$$

Die Berechnung erfolgt unter der Annahme konstanten Absolutdrucks während der Durchführung des erfindungsgemäßen Verfahrens.

Mit den Substitutionen $x_i = i \, V_{pump}$ und $y_i = P_i$ ist im Programmcode hinterlegt:

$$f(x_i) = P_{abs,amb} \cdot \frac{x_i}{V_{vessel,air,0} - x_i} \qquad \textbf{(3)}$$

Es werden n Pumphübe durchgeführt und n Wertepaare $(x_i, y_i)$ mit i = 1, ..., n ermittelt. Dabei bedeutet i = 1, ..., n den Laufindex für die Abfolge der Pumphübe, beginnend mit dem ersten Förderhub (i = 1), dem zweiten Förderhub (i = 2) bis zum letzten Förderhub (i = n), so dass insgesamt n Förderhübe ausgeführt werden. Die Wertepaare werden im Datenspeicher 120 gespeichert.

Mit der bekannten Ausgleichsrechnung der "Minimierung der Summe der Abstandsquadrate", auch unter der Bezeichnung "Methode der kleinsten Quadrate" (engl.: "method of least squares") bekannt, angewendet auf die Gleichung (2) zusammen mit Gleichung (3) werden die gesuchten Parameter auf der Grundlage von Gleichung (4) berechnet.

$$S = \sum_{i=1}^{n} (y_i - f(x_i))^2 = \sum_{i=1}^{n} \left( y_i - P_{abs,amb} \cdot \frac{x_i}{V_{vessel,air,0} - x_i} \right)^2 \qquad \textbf{(4)}$$

Die gesuchten Parameter $P_{abs,amb}$ und $V_{vessel,air,0}$ werden dabei nach der Methode der Minimierung der Summe der Abstandsquadrate so berechnet, dass die Fehlersumme S in Gleichung (4) ein Minimum annimmt. Die erste von zwei notwendigen Bedingungen dafür ergibt sich aus Gleichung (5):

$$\frac{\partial S}{\partial P_{abs,amb}} = \frac{\partial}{\partial P_{abs,amb}} \sum_{i=1}^{n} \left( y_i - P_{abs,amb} \cdot \frac{x_i}{V_{vessel,air,0} - x_i} \right)^2 =$$

$$2 \cdot \sum_{i=1}^{n} \left( y_i - P_{abs,amb} \cdot \frac{x_i}{V_{vessel,air,0} - x_i} \right) \cdot \left( -\frac{x_i}{V_{vessel,air,0} - x_i} \right) = 0 \qquad (5)$$

Aus Gleichung 5 folgt durch Umformung:

$$\sum_{i=1}^{n} \left( y_i \cdot \frac{x_i}{V_{vessel,air,0} - x_i} \right) - P_{abs,amb} \cdot \sum_{i=1}^{n} \left( \frac{x_i}{V_{vessel,air,0} - x_i} \right)^2 = 0 \qquad (6)$$

Aus Gleichung (6) erhält man durch Umformung eine erste Bestimmungsgleichung (7) für die beiden gesuchten Parameter. Die Bestimmungsgleichung (7) ist im Programmcode implementiert.

$$P_{abs,amb} = \frac{\sum_{i=1}^{n} \left( y_i \cdot \frac{x_i}{V_{vessel,air,0} - x_i} \right)}{\sum_{i=1}^{n} \left( \frac{x_i}{V_{vessel,air,0} - x_i} \right)^2} \qquad (7)$$

Die zweite von zwei notwendigen Bedingungen ergibt sich gemäß Gleichung (8):

$$\frac{\partial S}{\partial V_{vessel,air,0}} = \frac{\partial}{\partial V_{vessel,air,0}} \sum_{i=1}^{n} \left( y_i - P_{abs,amb} \cdot \frac{x_i}{V_{vessel,air,0} - x_i} \right)^2 =$$

$$2 \cdot \sum_{i=1}^{n} \left( y_i - P_{abs,amb} \cdot \frac{x_i}{V_{vessel,air,0} - x_i} \right) \cdot \left( + \frac{P_{abs,amb} \cdot x_i}{\left( V_{vessel,air,0} - x_i \right)^2} \right) = 0 \qquad (8)$$

Aus Gleichung (8) erhält man eine zweite Bestimmungsgleichung (9) für die beiden gesuchten Parameter.

$$\sum_{i=1}^{n} \left( y_i \cdot \frac{x_i}{\left( V_{vessel,air,0} - x_i \right)^2} \right) - P_{abs,amb} \cdot \sum_{i=1}^{n} \frac{x_i^2}{\left( V_{vessel,air,0} - x_i \right)^3} = 0 \qquad (9)$$

Durch Einsetzen der Beziehung für den gesuchten Absolutdruck gemäß Gleichung (7) in die Gleichung (9) ergibt sich eine implizite Bestimmungsgleichung (10) für den gesuchten Parameter $V_{vessel,air,0}$. Die Bestimmungsgleichung (10) ist im Programmcode implementiert und wird auf bekannte Weise mit einem numerischen Lösungsverfahren gelöst. Das bekannte numerische Lösungsverfahren, beispielsweise ausgewählt aus Bisektionsverfahren (z.B. Intervallhalbierungsverfahren) oder Regula falsi oder Newton-Raphsonsches Verfahren, ist im Programmcode implementiert.

$$\sum_{i=1}^{n}\left(\frac{y_i \cdot x_i}{\left(V_{vessel,air,0} - x_i\right)^2}\right) \cdot \sum_{i=1}^{n}\left(\frac{x_i}{V_{vessel,air,0} - x_i}\right)^2$$

$$-\sum_{i=1}^{n}\left(\frac{y_i \cdot x_i}{V_{vessel,air,0} - x_i}\right) \cdot \sum_{i=1}^{n}\frac{x_i^2}{\left(V_{vessel,air,0} - x_i\right)^3} = 0 \qquad (10)$$

Aus Gleichung (7) erhält man durch Einsetzen des berechneten Parameters $V_{vessel,air,0}$ den zweiten Parameter $P_{abs,amb}$. Es wäre natürlich auch möglich, in umgekehrter Reihenfolge zuerst $P_{abs,amb}$ und danach $V_{vessel,air,0}$ zu ermitteln.

Der gesuchte Absolutdruck wird in dem Ausführungsbeispiel mit 978 hPa und das initiale Luftvolumen mit 242 ml berechnet. Es hat sich überraschend herausgestellt, dass die Genauigkeit und die Reproduzierbarkeit des ermittelten Absolutdrucks den Anforderungen der Genauigkeit und Reproduzierbarkeit an die Berechnung der Betriebsparameter sehr gut genügen, so dass keine direkte Messung des Absolutdrucks mit einem Absolutdruckmesser erforderlich ist.

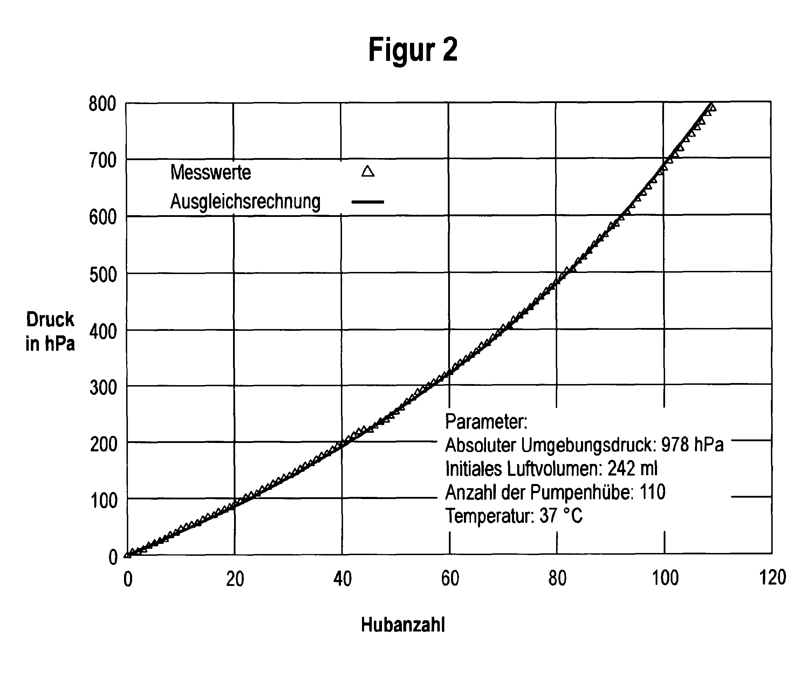

Figur 2 zeigt eine grafische Darstellung der Druckverhältnisse des Relativdrucks in der Mischkammer des Dialysierflüssigkeitssystems aus Figur 1 bei der Durchführung des erfindungsgemäßen Verfahrens. Die Anzahl der durchgeführten 110 Pumpenhübe ist auf der Abszisse aufgetragen. Der nach jedem Förderhub gemessene Relativdruck in der Mischkammer ist in Figur 2 in Abhängigkeit von der Anzahl der Pumpenhübe auf der Ordinate aufgetragen. Die einzelnen Wertepaare der Messwerte des Relativdrucks sind in Figur 2 durch Dreiecke dargestellt.

Die bei der Durchführung der Ausgleichsrechnung berechneten Parameter 978 hPa Absolutdruck und 242 ml initiales Luftvolumen in der Mischklammer 6 ergeben eingesetzt in die Gleichung 2 die in Figur 2 als durchgezogene Kurve dargestellte Ausgleichskurve. Mit anderen Worten werden von der Steuer- und Recheneinheit 110 mittels der Ausgleichsrechnung unter Verwendung aller Wertepaare die gesuchten Parameter Absolutdruck und initiales Luftvolumen so ermittelt, dass die Ausgleichskurve gemäß Gleichung (2) die Abhängigkeit des gemessenen Relativdrucks von der Anzahl der Förderhübe bestmöglich beschreibt. Zur Vermeidung von Missverständnissen wird darauf hingewiesen, dass der Absolutdruck aus der Kurve in Figur 2 natürlich nicht direkt abgelesen werden kann.

Die Steuer- und Recheneinheit 110 berechnet nach der Ermittlung des Absolutdrucks in einem weiteren Schritt den mindestens einen vom Absolutdruck abhängigen Betriebsparameter. Die für diesen weiteren Schritt erforderlichen Unterprogramme des erfindungsgemäßen Programmcodes sind dem Fachmann geläufig.

Im vorliegenden Ausführungsbeispiel berechnet die Steuer- und Recheneinheit 110 die gesuchte Siedetemperatur aufgrund der in der Steuer- und Recheneinheit hinterlegten Daten der Dampfdrucktabelle für Wasser oder aufgrund einer in der Steuer- und Recheneinheit hinterlegten Näherungsgleichung. Die Reinwassertemperatur am Heizstab im Dialysierflüssigkeitssystem wird so geregelt, dass sie unter der Siedetemperatur liegt. Besonders vorteilhaft wird die Reinwassertemperatur während der Heißdesinfektion stets rund 1,2 °C unter der Siedetemperatur geregelt. Die Steuer- und Recheneinheit 110 regelt den Aufheizvorgang des Reinwassers am Heizstab 17 mit Regeleingriffen 17a derart, dass die mittels des Temperatursensors 18 gemessene Temperatur des Reinwassers nach Passieren des Heizstabs 17 die berechnete Siedetemperatur um 1,2 °C unterschreitet und dadurch eine Dampfbildung sicher vermieden wird. Ein Benutzereingriff ist dafür nicht erforderlich. Ein Absolutdruckmesser wird erfindungsgemäß nicht benötigt. Die Regelung der Temperatur des Reinwassers erfolgt sehr genau. Die Sicherheit und Zuverlässigkeit des Dialysierflüssigkeitssystems wird dadurch verbessert.

Zur Einstellung des gewünschten Entgasungsdrucks verursacht die Steuer- und Recheneinheit 110 ausgehend vom Messwert des Absolutdrucks Regeleingriffe 19a an der Entgasungspumpe 19 derart, dass der beispielhaft geforderte Entgasungsdruck 150 hPa erreicht wird. Der Entgasungsdruck wird dazu mittels der drehzahlgeregelten Entgasungspumpe 19 als Druckabfall an der Entgasungsdrossel 20 eingestellt. Der Druckabfall an der Entgasungsdrossel 20 wird gemessen (Druckmessstellen in Figur 1 nicht gezeigt) und an die Steuer- und Recheneinheit 110 übertragen. Von der Steuer- und Recheneinheit 110 wird die Druckdifferenz vom berechneten Absolutdruck zum gewünschten Entgasungsdruck (also 978 hPa minus 150 hPa) berechnet und als Sollwert für die Regelung vorgegeben. Der Druckabfall an der Entgasungsdrossel 20 wird mit zwei Relativdrucksensoren (in Figur 1 nicht gezeigt) vor und nach der Entgasungsdrossel 20 gemessen und mit dem Sollwert der Druckdifferenz verglichen. Die Drehzahl der Entgasungspumpe 19 wird durch die Regeleingriffe 19a so geregelt, dass die erforderliche Druckdifferenz 828 hPa an der Entgasungsdrossel 20 abfällt und der gewünschte Entgasungsdruck erreicht wird. Ein Benutzereingriff ist dafür nicht erforderlich. Ein Absolutdruckmesser wird erfindungsgemäß nicht benötigt. Die Einstellung des Entgasungsdrucks erfolgt sehr genau. Die Sicherheit und Zuverlässigkeit des Dialysierflüssigkeitssystems wird dadurch verbessert.

Die Rechen- und Steuereinheit 110 speichert alle Ergebnisse in dem Speicher 120. Der Speicherinhalt des Datenspeichers 120 kann auf einem in Figur 1 nicht dargestellten Display der Blutbehandlungsvorrichtung angezeigt werden oder kann für Dokumentationszwecke über eine Datenschnittstelle aus dem Speicher ausgelesen werden.

Der Absolutdruck kann für den Benutzer sichtbar angezeigt werden, beispielsweise kann der Absolutdruck auf dem Bildschirm der Blutbehandlungsvorrichtung als numerischer Zahlenwert dargestellt werden.

Erfindungsgemäß gelingt die Lösung der Aufgabenstellungen der vorliegenden Erfindung mit dem gezeigten Ausführungsbeispiel. Die vorliegende Erfindung ist jedoch nicht auf das Ausführungsbeispiel beschränkt.

Bezugszeichenliste

| Bezugszeichen | Bezeichnung |
|---|---|
| 1 | Dialysierflüssigkeitssystem |
| 2 | Dialysator |
| 3 | semipermeable Membran |
| 4 | Blutkammer |
| 5 | Dialysierflüssigkeitskammer |
| 6 | Mischkammer |
| 7 | erste Leitung |
| 8 | erstes Fördermittel, Dosierkammer/ Membranpumpe |
| 8a | Steuereingriffe |
| 9 | zweite Leitung |
| 10 | zweites Fördermittel, Dosierpumpe / Membranpumpe |
| 10a | Steuereingriffe |
| 11 | dritte Leitung |
| 12 | drittes Fördermittel, Dosierpumpe / Membranpumpe |
| 12a | Steuereingriffe |
| 13 | Drucksensor |
| 14 | initiales Luftvolumen |
| 15 | Füllstandsmessvorrichtung |
| 16 | Initiales Flüssigkeitsvolumen |
| 17 | Heiz stab |
| 17a | Regeleingriff |
| 18 | Temperatursensor |
| 19 | Entgasungspumpe |
| 19a | Regeleingriff |
| 20 | Entgasungsdrossel |
| 21 | Leitung |
| 22 | Zahnradpumpe |
| 22a | Steuereingriffe |
| 23 | Bypass-Leitung |
| 24 | Absperrventil |
| 25 | Bilanziervorrichtung |
| 100 | zentrale Steuer- und Recheneinheit |
| 110 | Steuer- und Recheneinheit |
| 120 | Datenspeicher |

**Patentansprüche**

1. Verfahren zum Bestimmen mindestens eines vom Absolutdruck abhängigen Betriebsparameters einer Vorrichtung zur extrakorporalen Blutbehandlung mit den Schritten

   Einstellen des Umgebungsdrucks in einem zumindest teilweise mit einem initialen Luftvolumen (14) gefüllten geschlossenen Behälter (6) oder in einem geschlossenen, zumindest teilweise mit einem initialen Luftvolumen (14) und zumindest teilweise mit Flüssigkeit gefüllten Behälter (6),
   **gekennzeichnet dadurch, dass** das Verfahren zusätzlich folgende Schritte umfasst:

   Fördern einer Flüssigkeit in den zumindest teilweise mit einem initialen Luftvolumen (14) gefüllten geschlossenen Behälter (6) mittels einer Abfolge von mindestens zwei Förderhüben eines Fördermittels (8, 10,12) mit vorgegebenen Hubvolumina
   **oder**
   Fördern einer Flüssigkeit aus dem zumindest teilweise mit einem initialen Luftvolumen (14) und zumindest teilweise mit Flüssigkeit gefüllten Behälter (6) mittels einer Abfolge von mindestens zwei Förderhüben eines Fördermittels (22) mit vorgegebenen Hubvolumina
   Berechnen des insgesamt geförderten Volumens nach jedem Förderhub,
   Messen des Relativdrucks in dem Behälter (6) nach jedem Förderhub,
   Zuordnen des insgesamt geförderten Volumens nach jedem erfolgten Förderhub und des Messwerts des Relativdrucks in dem Behälter (6) nach jedem erfolgten Förderhub zu einem Wertepaar für den erfolgten Förderhub,
   Bestimmen des absoluten Umgebungsdrucks und bestimmen des initialen Luftvolumens (14) in dem Behälter (6) unter Verwendung der Wertepaare für die Abfolge der erfolgten Förderhübe,
   Berechnen mindestens eines vom absoluten Umgebungsdruck abhängigen Betriebsparameters der Vorrichtung zur extrakorporalen Blutbehandlung.

2. Verfahren nach Anspruch 1 **dadurch gekennzeichnet, dass** die mindestens zwei Förderhübe das gleiche Hubvolumen aufweisen und das Berechnen des insgesamt geförderten Volumens nach jedem Förderhub als Produkt aus der Anzahl der erfolgten Förderhübe und dem Hubvolumen erfolgt.

3. Verfahren nach Anspruch 1 mit dem zusätzlichen Schritt Hinterlegen des mindestens einen berechneten Betriebsparameters in einer Steuer- und Recheneinheit (110).

4. Verfahren nach einem der Ansprüche 1 bis 3 mit dem zusätzlichen Schritt Vorgeben des Hubvolumens eines Förderhubs als mindestens eine Größe ausgewählt aus der Gruppe:

   Betrag eines Volumens, Schrittanzahl, Schrittwinkel eines Schrittmotors und
   Förderdauer des Fördermittels.

5. Verfahren nach einem der Ansprüche 1 bis 4 **dadurch gekennzeichnet, dass** das Berechnen des absoluten Umgebungsdrucks und das Berechnen des initialen Luftvolumens (14) in dem Behälter (6) mit einem Gleichungssystem unter Verwendung des Gesetzes von Boyle-Mariotte für die Zustandsänderungen des Luftvolumens in dem Behälter (6), mit dem Gleichungssystem

$$P_i = P_{abs,amb} \cdot \frac{i \cdot V_{pump}}{V_{vessel,air,0} - i * V_{pump}}; \ i = 1,...,n$$

   durchgeführt wird, wobei in dem Gleichungssystem:

   $P_i$ den Relativdruck in dem Behälter (6) nach dem i-ten Förderhub, $P_{abs,amb}$ den Absolutdruck in der Umgebung,
   $V_{vessel,air,0}$ das initiale Luftvolumen in dem Behälter (6),
   $V_{pump}$ das Hubvolumen des Fördermittels,
   n die gesamte Anzahl der Förderhübe des Fördermittels,
   i den Laufindex für die Anzahl der Förderhübe
   bedeuten.

**6.** Verfahren nach Anspruch 5 **dadurch gekennzeichnet, dass** das Berechnen des absoluten Umgebungsdrucks und das Berechnen des initialen Luftvolumens in dem Behälter (6) durch Anwenden einer Ausgleichsrechnung auf das Gleichungssystem erfolgt.

**7.** Vorrichtung zum Bestimmen mindestens eines vom Absolutdruck abhängigen Betriebsparameters einer Vorrichtung zur extrakorporalen Blutbehandlung aufweisend
einen geschlossenen, zumindest teilweise mit einem initialen Luftvolumen (14) gefüllten Behälter (6)
**oder**
einen geschlossenen, zumindest teilweise mit einem initialen Luftvolumen (14) und zumindest teilweise mit Flüssigkeit gefüllten Behälter (6),
Mittel zum Einstellen des absoluten Umgebungsdrucks in dem Behälter (6)
ein Fördermittel (8, 10, 12, 22) zum Fördern einer Mehrzahl von Förderhüben einer Flüssigkeit in den Behälter (6) oder aus dem Behälter (6),
ein Mittel zum Messen des Relativdrucks (13) in dem Behälter (6),
eine Steuer- und Recheneinheit (110) ausgebildet zum
mindestens zweifachen Ausführen eines Förderhubs des Fördermittels, Messen des Relativdrucks in dem Behälter nach jedem Förderhub,
Auswerten des Relativdrucks in dem Behälter nach jedem Förderhub, Berechnen des insgesamt geförderten Volumens nach jedem Förderhub, Zuordnen des insgesamt geförderten Volumens nach jedem erfolgten Förderhub und des Relativdrucks in dem Behälter (6) nach jedem erfolgten Förderhub zu einem Wertepaar für den erfolgten Förderhub,
**gekennzeichnet dadurch, dass** die Steuer- und Recheneinheit zusätzlich folgende Schritte veranlasst:

Bestimmen des absoluten Umgebungsdrucks und Berechnen des initialen Luftvolumens in dem Behälter (6) unter Verwendung der Wertepaare für mindestens zwei erfolgte Förderhübe,
Berechnen mindestens eines vom absoluten Umgebungsdruck abhängigen Betriebsparameters der Vorrichtung zur extrakorporalen Blutbehandlung.

**8.** Vorrichtung nach Anspruch 7 **dadurch gekennzeichnet, dass** die Förderhübe das gleiche Hubvolumen aufweisen.

**9.** Vorrichtung nach einem der Ansprüche 7 bis 8 **dadurch gekennzeichnet, dass** die Steuer- und Recheneinheit (110) zusätzlich ausgebildet ist zum Hinterlegen des mindestens einen berechneten Betriebsparameters in einer Steuer- und Recheneinheit (100) der Vorrichtung zur extrakorporalen Blutbehandlung.

**10.** Vorrichtung nach einem der Ansprüche 7 bis 9 **dadurch gekennzeichnet, dass** die Steuer- und Recheneinheit (110) zusätzlich ausgebildet ist zum Vorgeben des Hubvolumens eines Förderhubs des Fördermittels (8, 10, 12) als mindestens eine Größe ausgewählt aus der Gruppe:

Betrag eines Volumens, Schrittanzahl, Schrittwinkel eines Schrittmotors und Förderdauer des Fördermittels (8, 10, 12).

**11.** Vorrichtung nach einem der Ansprüche 7 bis 10 **dadurch gekennzeichnet, dass** die Steuer- und Recheneinheit (110) zusätzlich ausgebildet ist zum Berechnen des absoluten Umgebungsdrucks und des initialen Luftvolumens in dem Behälter (6) mit einem Gleichungssystem unter Verwendung des Gesetzes von Boyle-Mariotte für die Zustandsänderungen des Luftvolumens in dem Behälter mit dem Gleichungssystem

$$P_i = P_{abs,amb} \cdot \frac{i \cdot V_{pump}}{V_{vessel,air,0} - i * V_{pump}}; \ i = 1, \ldots, n$$

wobei in dem Gleichungssystem:

$P_i$ den Relativdruck in dem Behälter (6) nach dem i-ten Förderhub,
$P_{abs,amb}$ den Absolutdruck in der Umgebung,
$V_{vessel,air,0}$ das initiale Luftvolumen in dem Behälter (6),
$V_{pump}$ das Hubvolumen des Fördermittels,
n die gesamte Anzahl der Förderhübe des Fördermittels,

i den Laufindex für die Anzahl der Förderhübe bedeuten.

**12.** Vorrichtung nach einem der Ansprüche 7 bis 11 **dadurch gekennzeichnet, dass** die Steuer- und Recheneinheit (110) zusätzlich ausgebildet ist zum Berechnen des absoluten Umgebungsdrucks und des initialen Luftvolumens in dem Behälter (6) durch Anwenden einer Ausgleichsrechnung auf das Gleichungssystem.

**13.** Vorrichtung nach einem der Ansprüche 7 bis 12 **dadurch gekennzeichnet, dass** das Fördermittel (8, 10,12) eine Pumpe ausgewählt aus der Gruppe Membranpumpe, Kolbenpumpe, Schlauchpumpe und Zahnradpumpe ist.

**14.** Blutbehandlungsvorrichtung aufweisend ein Dialysierflüssigkeitssystem (1) und eine Vorrichtung nach einem der Ansprüche 7 bis 13.

**15.** Vorrichtung nach Anspruch 14, **dadurch gekennzeichnet, dass** die Steuer- und Recheneinheit (110) der Vorrichtung zum Einstellen mindestens eines vom Absolutdruck abhängigen Betriebsparameters Teil der zentralen Steuer- und Recheneinheit (100) der Blutbehandlungsvorrichtung ist.

**16.** Vorrichtung nach einem der Ansprüche 14 oder 15 **dadurch gekennzeichnet, dass** der Behälter (6) eine Mischkammer (6) des Dialysierflüssigkeitssystems (1) der Hämodialysevorrichtung oder Hämodiafiltrationsvorrichtung ist.

**17.** Vorrichtung nach Anspruch 16 **dadurch gekennzeichnet, dass** das Fördermittel (8, 10, 12) eine Dosierpumpe ist zum Dosieren von Reinwasser in die Mischkammer
oder eine Dosierpumpe zum Dosieren eines Flüssigkonzentrats in die Mischkammer (6).

**18.** Computer-Programm-Produkt mit einem auf einem maschinenlesbaren Träger gespeicherten Programmcode zum Abspeichern in der Steuer- und Recheneinheit (110) einer Vorrichtung nach Anspruch 7 zur Veranlassung der maschinellen Schritte des Verfahrens nach einem der Ansprüche 1 bis 6, wenn der Programmcode in der Steuer- und Recheneinheit (110) abläuft.

## Claims

**1.** A method for determining at least one operating parameter of a device for extracorporeal blood treatment, wherein the parameter depends on the absolute pressure, comprising the steps:

adjusting the ambient pressure in a closed container (6) filled at least partially with an initial air volume (14) or in a closed container (6) filled at least partially with an initial air volume (14) and at least partially with liquid, **characterized in that** the method additionally comprises the following steps:

pumping a liquid into the closed container (6), which is filled at least partially with an initial air volume (14), by means of a sequence of at least two delivery strokes of a pumping means (8, 10, 12) with predefined displaced volumes,
or
pumping a liquid out of the container (6), which is filled at least partially with an initial air volume (14) and at least partially with liquid by means of a sequence of at least two delivery strokes of a pumping means (22) with predefined displaced volumes,
calculating the total volume pumped after each delivery stroke,
measuring the relative pressure in the container (6) after each delivery stroke,
assigning the total volume pumped after each delivery stroke effected and the measured value of the relative pressure in the container (6) after each delivery stroke effected to a value pair for the delivery stroke effected,
determining the absolute ambient pressure and determining the initial air volume (14) in the container (6) using the value pairs for the sequence of delivery strokes effected,
calculating at least one operating parameter of the device for extracorporeal blood treatment, wherein the operating parameter depends on the absolute ambient pressure.

**2.** The method according to Claim 1, **characterized in that** the at least two delivery strokes have the same displaced volume and the total volume pumped is calculated after each delivery stroke as the product of the number of delivery strokes effected and the displaced volume.

**3.** The method according to Claim 1, comprising the additional step:

> storing the at least one calculated operating parameter in a control and calculation unit (110).

**4.** The method according to any one of Claims 1 to 3, comprising the additional step:

> preselecting the displaced volume of a delivery stroke as at least one variable selected from the group:

>> amount of a volume, a number of steps, a stepping angle of a stepping motor and the pumping time of the pumping means.

**5.** The method according to any one of Claims 1 to 4, **characterized in that** the calculation of the absolute ambient pressure and the calculation of the initial air volume (14) in the container (6) are performed using an equation system, which uses the Boyle-Mariotte law for the changes in state of the air volume in the container (6), with the equation system:

$$P_i = P_{abs,amb} \cdot \frac{i \cdot V_{pump}}{V_{vessel,air,0} - i^* V_{pump}}; \quad i = 1, ..., n$$

wherein the following meanings are used in the equation system:

> $P_i$ denotes the relative pressure in the container (6) after the i-th delivery stroke,
> $P_{abs,amb}$ denotes the absolute ambient pressure,
> $V_{vessel,air,0}$ denotes the initial air volume in the container (6),
> $V_{pump}$ denotes the displaced volume of the pumping means,
> n denotes the total number of delivery strokes of the pumping means,
> i denotes the running index for the number of delivery strokes.

**6.** The method according to Claim 5, **characterized in that** the absolute ambient pressure and the initial air volume in the container (6) are calculated by applying a regression calculation to the equation system.

**7.** A device for determining at least one operating parameter of a device for extracorporeal treatment of blood, wherein the parameter depends on the absolute pressure, comprising:

> a closed container (6) filled at least partially with an initial air volume (14),
> or
> a closed container (6) filled at least partially with an initial air volume (14) and at least partially with liquid,
> means for adjusting the absolute ambient pressure in the container (6),
> pumping means (8, 10, 12, 22) for pumping a plurality of delivery strokes of a liquid into the container (6) or out of the container (6),
> a means for measuring the relative pressure (13) in the container (6),
> a control and calculation unit (110) designed for
> executing a delivery stroke of the pump means at least twice,
> measuring the relative pressure in the container after each delivery stroke,
> evaluating the relative pressure in the container after each delivery stroke,
> calculating the total volume pumped after each delivery stroke,
> assigning the total volume pumped after each delivery stroke effected and the relative pressure in the container (6) after each delivery stroke effected to a value pair for the delivery stroke effected,
> **characterized in that** the control and calculation unit additionally effectuates the following steps:

>> determining the absolute ambient pressure and calculating the initial air volume in the container (6) using the value pairs for at least two delivery strokes effected,
>> calculating at least one operating parameter of the device for extracorporeal blood treatment, wherein the parameter depends on the absolute ambient pressure.

**8.** The device according Claim 7, **characterized in that** the delivery strokes have the same displaced volume.

9. The device according to any one of Claims 7 to 8, **characterized in that** the control and computation unit (110) is additionally designed for
storing the at least one calculated operating parameter in a control and computation unit (100) of the device for extracorporeal blood treatment.

10. The device according to any one of Claims 7 to 9, **characterized in that** the control and computation unit (110) is additionally designed for predefining the displaced volume of a delivery stroke of the pump means (8, 10, 12) as at least one variable selected from the group:

absolute value of a volume, number of steps, stepping angle of a stepping motor and pumping period of the pump means (8, 10, 12).

11. The device according to any one of Claims 7 to 10, **characterized in that** the control and computation unit (110) is additionally designed for calculating the absolute ambient pressure and the initial air volume in the container (6) with an equation system using the Boyle-Mariotte law for the changes in state of the air volume in the container with the equation system:

$$P_i = P_{abs,amb} \cdot \frac{i \cdot V_{pump}}{V_{vessel,air,0} - i * V_{pump}} \; ; \; i = 1, ..., n$$

wherein, in the equation system:

$P_i$ is the relative pressure in the container (6) after the i-th delivery stroke,
$P_{abs,amb}$ is the absolute pressure in the environment,
$V_{vessel,air,0}$ is the initial air volume in the container (6),
$V_{pump}$ is the displaced volume of the pump means,
n is the total number of delivery strokes of the pump means,
i is the running index for the number of delivery strokes.

12. The device according to any one of Claims 7 to 11, **characterized in that** the control and computation unit (110) is additionally designed for calculating the absolute ambient pressure and the initial air volume in the container (6) by applying a regression calculation to the equation system.

13. The device according to any one of Claims 7 to 12, **characterized in that** the pump means (8, 10, 12) is a pump selected from the group including diaphragm pump, piston pump, hose pump and gear pump.

14. A blood treatment device, comprising a dialysis fluid system (1) and a device according to any one of Claims 7 to 13.

15. The device according to Claim 14, **characterized in that** the control and computation unit (110) of the device for adjusting at least one operating parameter that depends on the absolute pressure is part of the central control and computation unit (100) of the blood treatment device.

16. The device according to any one of Claims 14 or 15, **characterized in that** the container (6) is a mixing container (6) of the dialysis fluid system (1) of the hemodialysis device or hemodiafiltration device.

17. The device according to Claim 16, **characterized in that**
the pump means (8, 10, 12) is a metering pump for metering pure water into the mixing chamber
or a metering pump for metering a liquid concentrate into the mixing chamber (6).

18. A computer program product having a program code stored on a machine-readable carrier for storage in the control and computation unit (110) of a device according to Claim 7, for effectuating the machine steps of the method according to any one of Claims 1 to 6 when the program code is running in the control and computation unit (110).

**Revendications**

1. Procédé de détermination d'au moins un paramètre de fonctionnement dépendant d'une pression absolue d'un dispositif de traitement extracorporel du sang comprenant les étapes

   de réglage de la pression environnante dans un récipient (6) fermé rempli au moins partiellement d'un volume d'air initial (14) ou dans un récipient (6) fermé rempli au moins partiellement d'un volume d'air initial (14) et rempli au moins partiellement de liquide,
   **caractérisé en ce que** le procédé comprend les étapes supplémentaires suivantes :

   transport d'un liquide dans le récipient (6) fermé rempli au moins partiellement d'un volume d'air initial (14) au moyen d'une suite d'au moins deux courses de transport d'un moyen de transport (8, 10, 12) avec des cylindrées prédéfinies
   ou
   transport d'un liquide de l'au moins un récipient (6) fermé rempli au moins partiellement d'un volume d'air initial (14) et rempli au moins partiellement de liquide au moyen d'une suite d'au moins deux courses de transport d'un moyen de transport (22) avec des cylindrées prédéfinies
   calcul du volume total transporté après chaque course de transport,
   mesure de la pression relative dans le récipient (6) après chaque course de transport,
   attribution du volume total transporté après chaque course de transport exécutée et de la valeur de mesure de la pression relative dans le récipient (6) après chaque course de transport exécutée à une paire de valeurs pour la course de transport exécutée,
   détermination de la pression environnante absolue et détermination du volume d'air initial (14) dans le récipient (6) en utilisant les paires de valeurs pour la suite des courses de transport exécutées,
   calcul d'au moins un paramètre de fonctionnement dépendant de la pression environnante absolue du dispositif de traitement extracorporel du sang.

2. Procédé selon la revendication 1, **caractérisé en ce que** les au moins deux courses de transport présentent la même cylindrée et le calcul du volume total transporté après chaque course de transport en tant que produit est effectué à partir du nombre de courses de transport exécutées et de la cylindrée.

3. Procédé selon la revendication 1, comprenant l'étape supplémentaire de
   dépôt de l'au moins un paramètre de fonctionnement calculé dans une unité de commande et de calcul (110).

4. Procédé selon l'une des revendications 1 à 3, comprenant l'étape supplémentaire de prédéfinition de la cylindrée d'une course de transport en tant qu'au moins une grandeur sélectionnée parmi le groupe :

   grandeur d'un volume, nombre de pas, angle de pas d'un moteur pas à pas et durée de transport du moyen de transport.

5. Procédé selon l'une des revendications 1 à 4, **caractérisé en ce que** le calcul de la pression environnante absolue et le calcul du volume d'air initial (14) dans le récipient (6) sont effectués avec un système d'équation en employant la loi de Boyle-Mariotte pour les changements d'état du volume d'air dans le récipient (6), avec le système d'équation :

$$P_i = P_{abs,amb} \cdot \frac{i \cdot V_{pump}}{V_{vessel,air,0} - i * V_{pump}} \; ; \; i = 1, ..., n$$

   sachant que dans le système d'équation :

   $P_i$ est la pression relative dans le récipient (6) après la ième course de transport,
   $P_{abs,amb}$ est la pression absolue dans l'environnement,
   $V_{vessel,air,0}$ est le volume d'air initial dans le récipient (6),
   $V_{pump}$ est la cylindrée du moyen de transport,
   $n$ est le nombre total des courses de transport du moyen de transport,
   $i$ est l'indice instantané pour le nombre de courses de transport.

6. Procédé selon la revendication 5, **caractérisé en ce que** le calcul de la pression environnante absolue et le calcul du volume d'air initial dans le récipient (6) sont effectués en employant un calcul de compensation sur le système d'équation.

7. Procédé de détermination d'au moins un paramètre de fonctionnement dépendant de la pression absolue d'un dispositif de traitement extracorporel du sang présentant

un récipient (6) fermé rempli au moins partiellement d'un volume d'air initial (14)

ou

un récipient (6) fermé rempli au moins partiellement d'un volume d'air initial (14) et rempli au moins partiellement de liquide,

des moyens pour régler la pression environnante absolue dans le récipient (6)

un moyen de transport (8, 10, 12, 22) pour faire avancer une pluralité de courses de transport d'un liquide dans le récipient (6) ou hors du récipient (6),

un moyen pour mesurer la pression relative (13) dans le récipient (6),

une unité de commande et de calcul (110) conçue pour exécuter au moins deux fois une course de transport du moyen de transport,

mesure de la pression relative dans le récipient après chaque course de transport,

évaluation de la pression relative dans le récipient après chaque course de transport,

calcul du volume total transporté après chaque course de transport,

attribution du volume total transporté après chaque course de transport exécutée et de la pression relative dans le récipient (6) après chaque course de transport exécutée à une paire de valeurs pour la course de transport exécutée,

**caractérisé en ce que** l'unité de commande et de calcul déclenche les étapes supplémentaires suivantes :

détermination de la pression environnante absolue et calcul du volume d'air initial dans le récipient (6) en employant les paires de valeurs pour au moins deux courses de transport exécutées,

calcul d'au moins un paramètre de fonctionnement dépendant de la pression environnante absolue du dispositif de traitement extracorporel du sang.

8. Dispositif selon la revendication 7, **caractérisé en ce que** les courses de transport présentent la même cylindrée.

9. Dispositif selon l'une des revendications 7 à 8, **caractérisé en ce que** l'unité de commande et de calcul (110) est conçue en outre pour

déposer l'au moins un paramètre de fonctionnement calculé dans une unité de commande et de calcul (100) du dispositif de traitement extracorporel du sang.

10. Dispositif selon l'une des revendications 7 à 9, **caractérisé en ce que** l'unité de commande et de calcul (110) est en outre conçue pour

prédéfinir la cylindrée d'une course de transport du moyen de transport (8, 10, 12) en tant qu'au moins une grandeur sélectionnée parmi le groupe :

grandeur d'un volume, nombre de pas, angle de pas d'un moteur pas à pas et durée de transport du moyen de transport (8, 10, 12).

11. Dispositif selon l'une des revendications 7 à 10, **caractérisé en ce que** l'unité de commande et de calcul (110) est en outre conçue pour

calculer la pression environnante absolue et le volume d'air initial dans le récipient (6) avec un système d'équation en employant la loi de Boyle-Mariotte pour les changements d'état du volume d'air dans le récipient, avec le système d'équation :

$$P_i = P_{abs,amb} \cdot \frac{i \cdot V_{pump}}{V_{vessel,air,0} - i^* V_{pump}} \; ; \; i = 1,\ldots,n$$

sachant que dans le système d'équation :

$P_i$ est la pression relative dans le récipient (6) après la ième course de transport,

$P_{abs,amb}$ est la pression absolue dans l'environnement,

$V_{vessel,air,0}$ est le volume d'air initial dans le récipient (6),

$V_{pump}$ est la cylindrée du moyen de transport,

n est le nombre total des courses de transport du moyen de transport,

i est l'indice instantané pour le nombre de courses de transport.

12. Dispositif selon l'une des revendications 7 à 11, **caractérisé en ce que** l'unité de commande et de calcul (110) est en outre conçue pour
calculer la pression environnante absolue et le volume d'air initial dans le récipient (6) en employant un calcul de compensation sur le système d'équation.

13. Dispositif selon l'une des revendications 7 à 12, **caractérisé en ce que** le moyen de transport (8, 10, 12) est une pompe sélectionnée parmi le groupe de pompe à membrane, pompe à piston, pompe péristaltique et pompe à engrenages.

14. Dispositif de traitement du sang présentant un système de liquide de dialyse (1) et un dispositif selon l'une des revendications 7 à 13.

15. Dispositif selon la revendication 14, **caractérisé en ce que** l'unité de commande et de calcul (110) du dispositif pour régler au moins un paramètre de fonctionnement dépendant de la pression absolue fait partie de l'unité de commande et de calcul centrale (100) du dispositif de traitement du sang.

16. Dispositif selon l'une des revendications 14 ou 15, **caractérisé en ce que** le récipient (6) est une chambre de mélange (6) du système de liquide de dialyse (1) du dispositif d'hémodialyse ou du dispositif d'hémodiafiltration.

17. Dispositif selon la revendication 16, **caractérisé en ce que**
le moyen de transport (8, 10, 12) est une pompe de dosage pour doser de l'eau pure dans la chambre de mélange ou une pompe de dosage pour doser un concentré liquide dans la chambre de mélange (6).

18. Produit de programme informatique comprenant un code de programme enregistré sur un support lisible par machine pour le stockage dans l'unité de commande et de calcul (110) d'un dispositif selon la revendication 7 pour déclencher les étapes manuelles du procédé selon l'une des revendications 1 à 6 lorsque le code de programme est exécuté dans l'unité de commande et de calcul (110) .

Figur 1

# Figur 2

EP 2 717 938 B1

**EP 2 717 938 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20090124963 A1
- US 20090099498 A1
- DE 19919572 A1